# FASCICULE DE BREVET EUROPEEN

(11) **EP 2 718 006 B1**
(45) Date de publication et mention de la délivrance du brevet: **25.03.2015**
(21) Numéro de dépôt: 12731100.9
(22) Date de dépôt: 01.06.2012
(51) Int. Cl.: B01J 19/24, F28D 9/00

(54) **REACTEUR A PLAQUES AVEC INJECTION IN SITU**
PLATTENREAKTOR MIT IN-SITU-INJEKTION
PLATE-TYPE REACTOR WITH IN-SITU INJECTION

(30) Priorité: 06.06.2011 FR 1154885
(43) Date de publication de la demande: 16.04.2014
(73) Titulaire: Arkema France, 92700 Colombes (FR)
(72) Inventeur: DUBOIS, Jean-Luc, F-69390 Millery (FR)
(74) Mandataire: Bonnel, Claudine
(86) Numéro de dépôt international: PCT/FR2012/051233
(87) Numéro de publication internationale: WO 2012/168631

(56) Documents cités:
- EP-A1- 0 995 491
- DE-A1-102006 010 368
- US-A1- 2005 158 217

## Description

La présente invention concerne un réacteur chimique présentant une géométrie de type à plaques, permettant l'injection *in situ* d'une substance telle que l'oxygène dans le milieu réactionnel, éventuellement de manière multiétagée. L'invention concerne également un procédé de réaction chimique utilisant ledit réacteur.

### ARRIERE-PLAN TECHNIQUE

On connaît la mise en oeuvre de réactions chimiques, notamment impliquant une catalyse hétérogène, dans des réacteurs à lit fixe. Lorsque ces réactions chimiques sont fortement endothermiques ou fortement exothermiques, le contrôle de la chaleur absorbée ou émise par la réaction suppose que le réacteur dispose de surfaces d'échange thermique étendues.

Par exemple, une géométrie classique de réacteurs chimiques à lit fixe est la géométrie multitubulaire. Celle-ci présente notamment pour inconvénient d'impliquer des opérations de chargement et de déchargement de catalyseur relativement complexes et longues conduisant à des pertes de productivité. Par ailleurs, les réacteurs multitubulaires présentent un coût de fabrication très élevé, mais aussi un poids très élevé lié aux masses de métaux qu'il est nécessaire d'utiliser. Une autre contrainte sur ces réacteurs est liée à leur mode de fabrication et de transport : ils sont limités en taille car, fabriqués et éprouvés en usine, ils doivent ensuite être transportés jusqu'au lieu d'utilisation finale.

Une autre géométrie connue pour ces réacteurs est la géométrie à plaques. Dans un réacteur à plaques, les compartiments réactionnels sont délimités par des plaques d'échange thermique. Les documents EP 0995491 et EP 1147807 fournissent des exemples de tels réacteurs à plaques.

Un autre exemple figure dans le document US 2005/0020851, qui décrit un tel réacteur utilisé pour l'oxydation d'un précurseur C3 ou C4 en acroléine, méthacroléine, acide acrylique ou acide méthacrylique.

Le document US 2005/0158217 décrit également un réacteur de ce type, dans lequel des thermocouples sont disposés dans les compartiments réactionnels (sans les traverser) pour contrôler la réaction.

Le document US 2005/0226793 décrit une disposition particulière de plaques d'échange thermique, dans laquelle les saillies de chaque plaque font face aux renfoncements de la plaque adjacente et réciproquement, afin d'améliorer le contrôle de la température.

Enfin, le document US 2006/0276334 fournit un autre exemple de réacteur de ce type, dans lequel les plaques d'échange thermique comportent un revêtement (dépôt) fissuré.

L'ensemble des réacteurs ci-dessus présentent pour inconvénient de ne pas permettre un contrôle / une gestion suffisant(e) de la température dans le réacteur, en phase de réaction et / ou en phase de régénération.

Il existe donc un réel besoin de permettre un meilleur contrôle de la température dans les réacteurs à lit fixe.

### RESUME DE L'INVENTION

L'invention concerne en premier lieu un réacteur chimique comprenant une pluralité de plaques d'échange thermique définissant entre elles des compartiments réactionnels, dans lequel chaque plaque d'échange thermique comporte deux parois définissant entre elles au moins un espace d'échange thermique, les parois respectives étant fixées entre elles par des zones de jonction, et le réacteur comprend également au moins un dispositif d'injection de substance dans les compartiments réactionnels, ledit dispositif d'injection de substance traversant les plaques d'échange thermique dans des zones de jonction respectives de celles-ci.

Selon un mode de réalisation, le dispositif d'injection de substance est un dispositif d'injection de gaz, et de préférence un dispositif d'injection de gaz comprenant de l'oxygène.

Selon un mode de réalisation, le dispositif d'injection de substance est une conduite présentant une pluralité d'orifices d'injection.

Selon un mode de réalisation, les zones de jonction sont disposées sur les plaques d'échange thermique sous forme de bandes, de préférence parallèles, ou sous forme de points.

Selon un mode de réalisation, le réacteur chimique comporte une pluralité de dispositifs d'injection de substance, de préférence parallèles entre eux, et de préférence connectés à un système de distribution de substance.

Selon un mode de réalisation, les plaques d'échange thermique sont disposées dans une enceinte de manière radiale, ou de manière parallèle entre elles, et de préférence sont groupées en modules.

L'invention concerne également un procédé de réaction chimique, comprenant l'amenée de réactifs en entrée d'un compartiment réactionnel défini entre deux plaques d'échange thermique, le soutirage de produits de réaction en sortie du compartiment réactionnel, ainsi que l'injection d'une substance dans le compartiment réactionnel, dans lequel chaque plaque d'échange thermique comporte deux parois définissant entre elles au moins un espace d'échange thermique, les parois respectives étant fixées entre elles par des zones de jonction, et l'injection de substance étant effectuée au moyen d'au moins un dispositif d'injection de substance traversant les plaques d'échange thermique dans des zones de jonction respectives de celles-ci.

Selon un mode de réalisation, la réaction est du type catalytique hétérogène et dans lequel un catalyseur est disposé dans les compartiments réactionnels, de préférence sous forme de particules solides. Selon un mode de réalisation, le procédé comprend en alternance des phases de production et des phases de régénération du catalyseur, l'injection de substance étant effectuée lors des phases de réaction et / ou lors des phases de régénération.

Selon un mode de réalisation, la substance est un réactif ou un composé catalytique ou un composé apte à régénérer un catalyseur.

Selon un mode de réalisation, la substance est un gaz comprenant de l'oxygène.

Selon un mode de réalisation, la substance est injectée en plusieurs points du parcours des réactifs dans le compartiment réactionnel.

Selon un mode de réalisation, le procédé est mis en oeuvre dans un réacteur chimique tel que décrit ci-dessus.

Selon un mode de réalisation, le procédé est :
- un procédé de déshydratation du glycérol en acroléine ; ou
- un procédé de déshydratation de l'acide lactique en acide acrylique ; ou
- un procédé de déshydratation de l'acide 3-hydroxypropionique en acide acrylique ; ou
- un procédé de déshydratation de l'acide 3-hydroxyisobutyrique en acide méthacrylique ; ou
- un procédé de déshydratation de l'acide 2-hydroxyisobutyrique, encore appelé alpha-hyroxyisobutyrique, en acide methacrylique ; ou
- un procédé de préparation d'une hydrofluorooléfine ou d'un hydrofluorocarbure, de préférence un procédé de préparation d'un fluoropropène, et de manière tout particulièrement préférée un procédé de préparation du 2,3,3,3-tétrafluoropropène.

La présente invention permet de surmonter les inconvénients de l'état de la technique. Elle fournit plus particulièrement un réacteur à lit fixe, dans lequel le contrôle de la température en tout point du réacteur est amélioré.

Ceci est accompli grâce à une géométrie de réacteur à plaques, dans laquelle une injection d'une substance *in situ,* dans les compartiments réactionnels, est effectuée au moyen de dispositifs d'injection traversant les plaques. Cette injection permet de mieux contrôler les processus chimiques dans l'ensemble des compartiments réactionnels, en modifiant la composition du flux réactionnel ou les conditions de réaction *in situ.*

Selon certains modes de réalisation particuliers, l'invention présente également une ou de préférence plusieurs des caractéristiques avantageuses énumérées ci-dessous.
- L'invention est particulièrement appropriée pour la mise en oeuvre de réactions dans lesquelles le catalyseur est susceptible de désactivation par cokage, et doit être régénéré par combustion. En effet, en choisissant un gaz comprenant de l'oxygène en tant que substance injectée *in situ,* on peut effectuer la régénération de façon beaucoup plus rapide, avec une meilleure homogénéité de température dans le réacteur. On limite par ailleurs les risques d'explosion ou d'emballement, en évitant une pression partielle élevée d'oxygène en entrée du réacteur.
- L'invention permet de réaliser un gain de rendement de l'ordre de quelques pourcents. L'invention permet aussi de limiter les temps d'arrêt du réacteur pour régénération et donc d'obtenir un gain de productivité typiquement de l'ordre de 25 à 100 %.

### BREVE DESCRIPTION DES FIGURES

La **figure 1** représente en perspective et en vue éclatée une partie d'un réacteur selon un mode de réalisation de l'invention.
Les **figures 2** et **3** représentent en perspective et en coupe des détails d'une plaque d'échange thermique dans un réacteur selon un mode de réalisation de l'invention.
La **figure 4** représente une coupe d'une partie d'un réacteur selon un mode de réalisation de l'invention.
La **figure 5** représente en perspective et en coupe un détail d'une plaque d'échange thermique dans un réacteur selon un autre mode de réalisation de l'invention.

### DESCRIPTION DE MODES DE REALISATION DE L'INVENTION

L'invention est maintenant décrite plus en détail et de façon non limitative dans la description qui suit.

### Description générale du réacteur

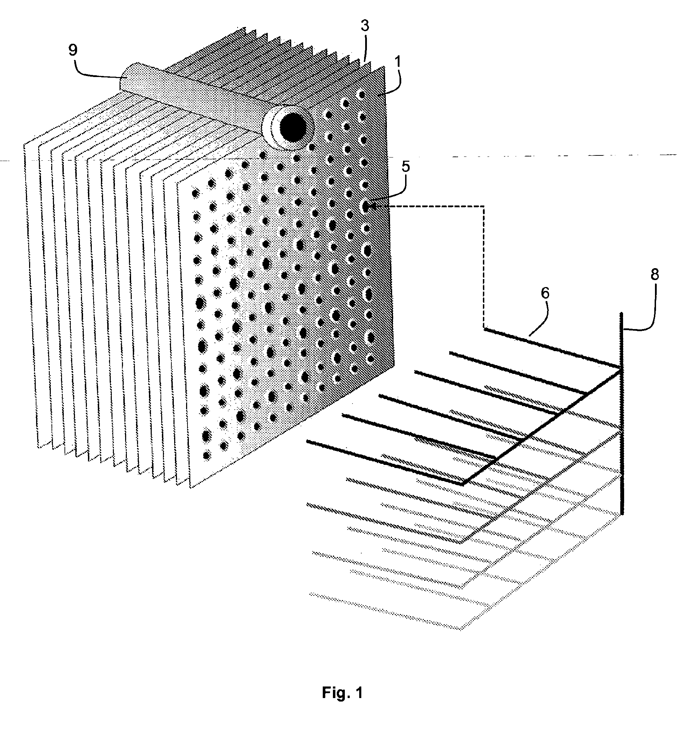

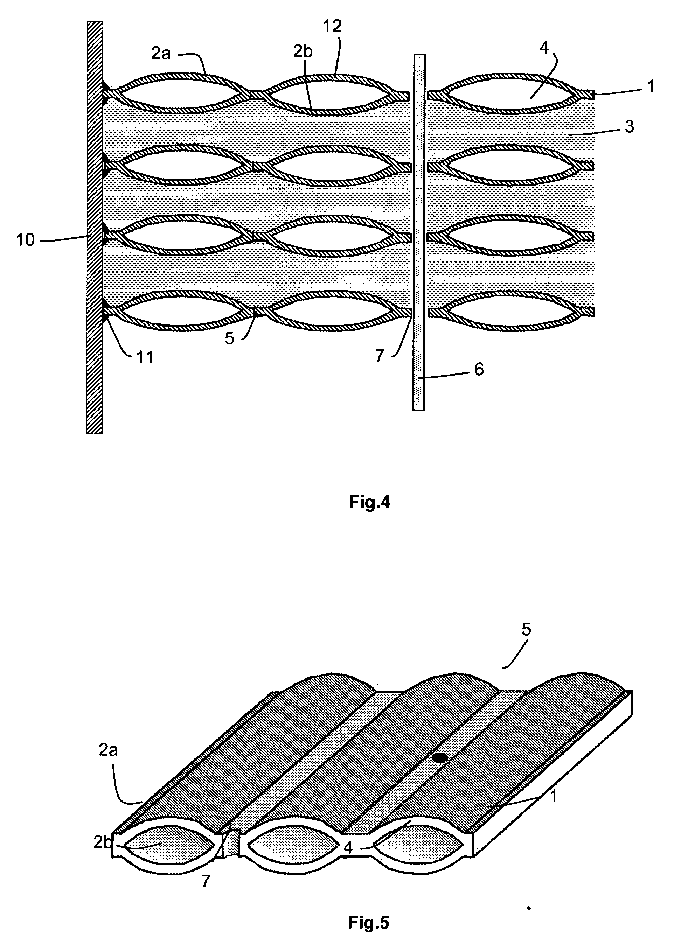

En faisant référence principalement aux **figures 1** et **4****,** de manière générale, le réacteur selon l'invention comprend une enceinte, qui est avantageusement essentiellement cylindrique à section circulaire, à l'intérieur de laquelle sont disposées un ensemble de plaques d'échange thermique **1** délimitant entre elles des compartiments réactionnels 3.

Les compartiments réactionnels 3 sont avantageusement remplis avec un catalyseur approprié pour la réaction mise en oeuvre, ce catalyseur étant de préférence sous forme de particules solides (billes, grains ou poudre) ou sous forme de monolithe poreux ou de blocs de monolithe poreux.

Le réacteur peut fonctionner selon au moins deux modes, à savoir un mode de production, au cours duquel la réaction chimique visée a lieu dans les compartiments réactionnels ; et un mode de régénération, au cours duquel le catalyseur au moins partiellement désactivé est régénéré.

Dans le cadre de la présente demande, l'expression « flux réactionnel » est comprise au sens large et désigne un flux traversant les compartiments réactionnels 3, étant entendu que la composition du flux réactionnel varie entre l'entrée et la sortie des compartiments réactionnels 3.

En phase de production, le flux réactionnel en entrée des compartiments réactionnels 3 comprend tout ou partie des réactifs (une autre partie des réactifs pouvant éventuellement être apportée par les dispositifs d'injection de substance décrits ci-dessous), et le flux réactionnel en sortie des compartiments réactionnels 3 comprend au moins en partie les produits de la réaction.

En phase de régénération, le flux réactionnel en entrée des compartiments réactionnels 3 peut comprendre un ou plusieurs composés aptes à régénérer le catalyseur, et le flux réactionnel en sortie des compartiments réactionnels 3 comprend des résidus de la régénération. Alternativement, le flux réactionnel en entrée des compartiments réactionnels 3 peut être un simple flux inerte (les composés aptes à régénérer le catalyseur pouvant être injectés au moyen des dispositifs d'injection de substance 6 décrits ci-dessous).

Par rapport à une géométrie multitubulaire, une géométrie à plaques présente une plus grande simplicité de fabrication, d'utilisation et de maintenance.

Chaque plaque d'échange thermique 1 définit un plan moyen. On désigne par « longueur » la dimension des plaques 1 dans ce plan selon l'axe principal de l'enceinte du réacteur (axe cylindrique en général) et par « largeur » la dimension des plaques 1 orthogonale à la précédente dans ce même plan.

Généralement, l'axe de l'enceinte du réacteur est disposé selon la direction verticale. Cela implique que la longueur des plaques 1 est selon la direction verticale, et que la largeur et l'épaisseur des plaques 1 sont selon des directions horizontales.

La largeur des plaques 1 est généralement dictée par des considérations de fabrication ; elle peut par exemple être de 100 à 2500 mm, et en particulier de 500 à 1500 mm. La longueur des plaques 1 dépend de la réaction et en particulier de son profil de température. Elle peut par exemple être de 500 à 7000 mm, et en particulier de 3000 à 4000 mm.

Selon un mode de réalisation, les plaques d'échange thermique 1 sont disposées selon un arrangement radial dans l'enceinte. Un tel arrangement radial est par exemple décrit dans le document US 2005/0226793, en relation notamment avec la figure 6. Cet arrangement permet une utilisation optimale de la totalité de l'enceinte du réacteur, lorsque celle-ci est cylindrique à section circulaire.

Selon un mode de réalisation alternatif au précédent, et qui est préféré afin de simplifier et d'uniformiser l'écoulement du flux réactionnel, les plaques d'échange thermique 1 sont disposées essentiellement parallèlement les unes aux autres, de sorte que les compartiments réactionnels 3 sont des volumes d'épaisseur globalement uniforme (à la déformation locale des plaques d'échange thermique 1 près).

Dans ce cas, afin de permettre une meilleure utilisation de l'espace disponible dans l'enceinte, il peut être avantageux de répartir les plaques d'échange thermique 1 et les compartiments réactionnels 3 intercalaires en modules réactionnels de forme parallélépipédique rectangle. Un tel arrangement des plaques d'échange thermique 1 en modules est décrit par exemple dans le document US 2005/0020851, notamment en relation avec les figures 1A, 1C, 1D, 1E et 1F.

Une telle structure modulaire peut permettre d'adapter le réacteur de manière flexible vis-à-vis de la capacité requise, par exemple en installant uniquement une partie des modules dans le réacteur pour en moduler la capacité, ou en fermant certains modules pour les isoler du flux réactionnel.

Les modules peuvent être de mêmes dimensions, ce qui en simplifie la fabrication. Ils peuvent également être de dimensions différentes, afin d'optimiser l'occupation de l'espace dans l'enceinte du réacteur.

De préférence, les modules peuvent être retirés, remplacés ou échangés de manière individuelle, ce qui simplifie les opérations de maintenance.

Chaque module est de préférence maintenu en position et stabilisé dans l'enceinte par un guide approprié, par exemple un cadre rectangulaire 10 auquel les plaques 1 du module peuvent être fixées par des moyens de fixation 11. Les guides ou cadres 10 des différents modules peuvent être scellés entre eux pour éviter tout flux d'un module à l'autre.

L'espace intermédiaire situé entre la paroi de l'enceinte et le ou les modules adjacents peut être séparé des compartiments réactionnels 3 par scellement (et éventuellement par remplissage par des matériaux inertes et par pressurisation), afin d'éviter toute réaction ou accumulation de matière dans cette zone.

Différents modes possibles de mise en place des modules dans l'enceinte et de scellement de l'ensemble sont décrits en détail dans le document US 2005/0020851.

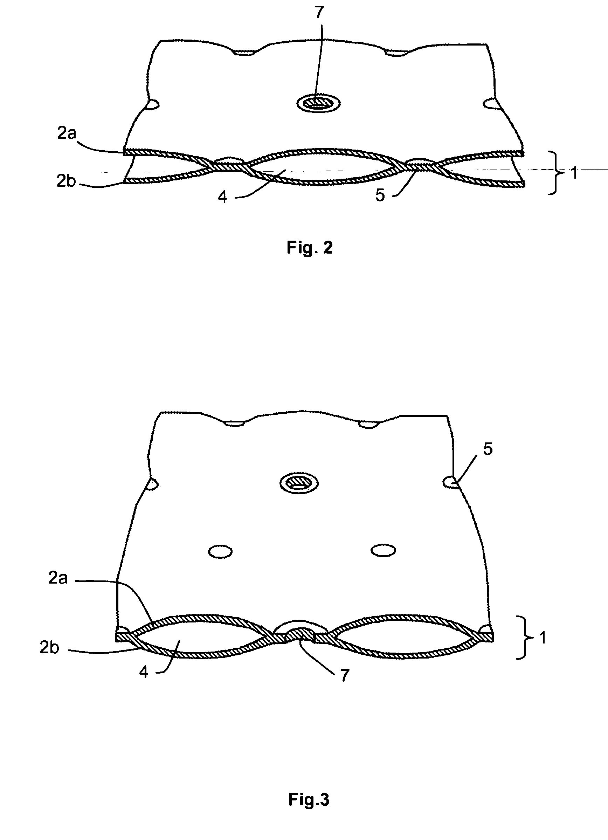

En faisant référence principalement aux **figures 2** à **5****,** chaque plaque d'échange thermique 1 est formée de deux parois 2a, 2b entre lesquelles est ménagé au moins un espace d'échange thermique 4. Les parois 2a, 2b peuvent par exemple être constituées de feuilles d'acier inoxydable, généralement de forme rectangulaire. L'épaisseur des parois 2a, 2b peut être par exemple de 1 à 4 mm, notamment de 1,5 à 3 mm et particulièrement de 2 à 2,5 mm.

Les deux parois 2a, 2b sont fixées l'une à l'autre par des zones de jonction 5. Cette fixation peut être effectuée par exemple par soudure des parois 2a, 2b entre elles. Les zones de jonction 5 peuvent être de toute forme.

Selon un mode de réalisation, les zones de jonction 5 sont sous forme de points (comme c'est le cas sur les **figures 1** à **4****).**

Par « points » on entend ici des zones de jonction 5 présentant une dimension relativement faible par rapport à la dimension totale de la plaque d'échange thermique 1, et ce aussi bien dans le sens de la longueur que dans le sens de la largeur de la plaque d'échange thermique 1.

A titre d'exemple, chaque zone de jonction 5 de type point présente une largeur inférieure ou égale à 5 % ou à 1 % ou à 0,5 % de la largeur totale de la plaque, ainsi qu'une longueur inférieure ou égale à 5 % ou à 1 % ou à 0,5 % de la longueur totale de la plaque 1.

Ainsi, les zones de jonction 5 dites en forme de points peuvent avoir de préférence une forme circulaire / discale, mais également éventuellement une forme polygonale (par exemple de carrés).

Lorsque les zones de jonction 5 sont sous forme de « points » au sens de la présente demande, ces points peuvent être répartis selon un réseau bidimensionnel sur chaque plaque d'échange thermique 1, par exemple un réseau à maille rectangulaire ou à maille carrée ; ou selon un pavage hexagonal par exemple.

Selon un mode de réalisation particulier, les zones de jonction 5 en forme de points sont réparties en lignes parallèles, l'espacement entre les zones de jonction successives au sein d'une même ligne étant par exemple de 30 à 80 mm, ou de 35 à 70 mm, ou de 40 à 60 mm. Et les lignes de points sont de préférence équidistantes, avec une séparation entre lignes par exemple de 5 à 80 mm, ou de 8 à 70 mm, ou de 10 à 60 mm. Les lignes de points successives peuvent être décalées les unes par rapport aux autres selon la direction des lignes, par exemple d'une moitié d'espacement entre points d'une même ligne. Autrement dit, dans ce cas, les points des lignes sont en alignement orthogonal aux lignes à raison d'une ligne sur deux.

Selon un autre mode de réalisation représenté à la **figure 5****,** les zones de jonction 5 sont sous forme de bandes, c'est-à-dire de lignes. Les bandes peuvent être courbes, ou de préférence droites. Les bandes adjacentes (successives) sont de préférence parallèles entre elles, et par exemple équidistantes. Ces bandes peuvent par exemple être disposées dans la direction de la longueur des plaques d'échange thermique 1 ou selon leur largeur. A titre d'exemple, la distance entre bandes peut être de 100 à 1500 et de préférence de 400 à 1000 mm quand elles sont arrangées dans le sens de la longueur du réacteur, et de 5 à 2000 mm et de préférence de 40 à 120 mm quand elles sont arrangées dans le sens de la largeur du réacteur.

Lorsque les zones de jonction 5 sont sous forme de points, l'espace d'échange thermique 4 entre les parois 2a, 2b de la plaque d'échange thermique 1 est généralement un espace unique. En revanche, lorsque les zones de jonction 5 sont sous forme de bandes, l'espace d'échange thermique 4 entre les parois 2a, 2b de la plaque d'échange thermique 1 est généralement composé d'une pluralité de canaux délimités par des zones de jonction 5 successives.

Il faut noter qu'il est possible de combiner des zones de jonction 5 sous forme de points avec des zones de jonction 5 sous forme de bandes.

En plus des zones de jonctions 5 décrites ci-dessus, les parois 2a, 2b de chaque plaque d'échange thermique 1 sont avantageusement jointes entre elles ou scellées à leur périphérie, afin d'obtenir un confinement du fluide caloporteur dans l'espace d'échange thermique 4. De préférence, cette jonction ou ce scellement n'est pas total, afin de ménager au moins une ouverture pour l'entrée du fluide caloporteur dans l'espace d'échange thermique 4 et au moins une ouverture pour la sortie du fluide caloporteur.

Généralement, on fait circuler un fluide caloporteur dans l'espace d'échange thermique 4 de chaque plaque 1 afin d'apporter de la chaleur aux compartiments réactionnels 3 (cas d'une réaction endothermique) ou afin d'évacuer de la chaleur des compartiments réactionnels (cas d'une réaction exothermique). Le fluide caloporteur peut également être une huile minérale ou une huile de synthèse, par exemple un Jarytherm ou un Dowtherm. Le fluide caloporteur peut aussi être un mélange de nitrates et / ou de nitrites fondus, par exemple un mélange eutectique de nitrate de sodium, nitrate de potassium, nitrite de sodium, ayant un point de fusion bas et une capacité calorifique élevée. De préférence, le fluide caloporteur est utilisé à des fins d'évacuation de la chaleur.

Le fluide caloporteur peut être notamment une solution aqueuse comportant éventuellement des additifs tels que de l'hydrazine ou de l'ammoniac ou un inhibiteur de corrosion. La circulation du fluide caloporteur dans les espaces d'échange de chaleur 4 peut être de type co-courant ou contre-courant ou transversal (croisé) par rapport à la circulation du flux réactionnel dans les compartiments réactionnels adjacents 3.

Pour un transfert de chaleur optimal, le fluide caloporteur change en partie d'état lors de son passage dans l'espace d'échange de chaleur 4, par exemple il passe au moins en partie de l'état liquide à l'état vapeur en cas de réaction exothermique.

Pour permettre la présence du fluide caloporteur dans les espaces d'échange de chaleur 4, les parois 2a, 2b des plaques d'échange thermique 1 sont déformées (gonflées) à distance des zones de jonction 5. En d'autres termes, chaque plaque d'échange thermique 1, en utilisation, comporte des portions d'épaisseur minimale (ou dépressions) au niveau des zones de jonction 5 des parois 2a, 2b, et des portions d'épaisseur maximale (ou protubérances 12) à distance des zones de jonction 5 des parois 2a, 2b.

Il est possible de disposer les zones de jonction 5 sur les plaques 1, et d'aligner les plaques d'échange thermique 1 successives, de telle sorte que les protubérances 12 des plaques 1 respectives soient alignées et que les dépressions des plaques 1 respectives soient alignées orthogonalement aux plaques. Un tel arrangement facilite la mise en place des dispositifs d'injection de substance 6 décrits ci-dessous, de manière perpendiculaire aux plaques 1.

Mais il est également possible de disposer les zones de jonction 5 sur les plaques 1, et d'aligner les plaques d'échange thermique 1 successives, de telle sorte que les protubérances 12 des plaques 1 respectives soient décalées, et que les dépressions des plaques 1 respectives soient décalées. Par exemple les protubérances 12 de chaque plaque 1 peuvent être alignées avec les dépressions de sa ou ses plaques 1 voisines orthogonalement aux plaques 1 et réciproquement. Un tel arrangement permet de réduire les variations d'épaisseur des compartiments réactionnels 3. Le document US 2005/0226793 fournit un exemple d'arrangement de ce type. Dans le cadre de l'invention, cet arrangement nécessite toutefois que les dispositifs d'injection de substance 6 décrits ci-dessous soient disposés de manière inclinée par rapport à la direction orthogonale aux plaques 1, ce qui peut compliquer la conception du réacteur.

Typiquement, l'épaisseur minimale des compartiments réactionnels 3 (c'est-à-dire l'épaisseur au point ou aux points où les plaques adjacentes sont les moins espacées) est de 8 à 150 mm, par exemple de 10 à 100 mm, notamment de 12 à 50 mm, en particulier de 14 à 25 mm, et à titre d'illustration de 16 à 20 mm. En tout état de cause, l'épaisseur des compartiments réactionnels 3 doit être adaptée pour pouvoir remplir les compartiments réactionnels 3 avec le catalyseur, qui est par exemple sous forme de grains. Les grains de catalyseur ont généralement une dimension (Dv50) de 1 à 10 mm, par exemple de 5 mm environ. On souhaite que l'épaisseur des compartiments réactionnels 3 soit de l'ordre de 3 à 5 grains au moins, pour pouvoir les remplir facilement. On peut aussi utiliser des formes de catalyseur en blocs monolithiques de 20 mm d'épaisseur par exemple.

Il est également possible de prévoir des compartiments réactionnels 3 d'épaisseurs différentes au sein du réacteur.

Les compartiments réactionnels 3 peuvent être isolés les uns par rapport aux autres par scellement, ou bien ils peuvent communiquer par leurs extrémités.

Des éléments d'espacement peuvent être prévus entre les plaques d'échange thermique 1 successives, afin de limiter les déformations de plaques 1 et les déplacements relatifs des plaques 1.

Un système de distribution de fluide caloporteur et un système de collecte de fluide caloporteur sont prévus de part et d'autre des plaques d'échange thermique 1. On peut par exemple prévoir un système de distribution et un système de collecte par module.

Le fluide caloporteur peut circuler de façon naturelle ou forcée dans les espaces d'échange thermique 4. Dans ce deuxième cas, on peut prévoir des moyens de pompage en entrée.

Des compensateurs de dilatation thermique peuvent être prévus sur l'enceinte (en périphérie et / ou aux extrémités de celle-ci). Les systèmes de distribution susmentionnés présentent avantageusement des portions courbes ou anguleuses permettant de compenser la dilatation thermique.

Un système de distribution de flux réactionnel 9 et un système de collecte de flux réactionnel (non représenté) sont prévus de part et d'autres des compartiments réactionnels 3. On peut par exemple prévoir un système de distribution et un système de collecte par module. Le flux réactionnel peut être de type liquide ou gazeux ou mixte. Le flux réactionnel peut circuler de haut en bas ou de bas en haut par rapport à l'enceinte.

Des moyens de rétention de catalyseur (typiquement des plaques perforées) sont prévus en entrée et en sortie des compartiments réactionnels 3.

II est possible de prévoir un chicanage dans les compartiments réactionnels 3 et / ou dans l'espace d'échange thermique 4, afin de forcer l'écoulement du fluide caloporteur et / ou du flux réactionnel selon un parcours déterminé.

### Injection de substance in situ dans les compartiments réactionnels

L'invention prévoit au moins un dispositif d'injection de substance 6 dans les compartiments réactionnels 3, de préférence une pluralité. Les dispositifs d'injection de substance 6 traversent les plaques d'échange thermique 1 et les compartiments réactionnels 3 entre les plaques d'échange thermique 1.

Selon le mode de réalisation illustré dans les figures, qui est particulièrement simple à mettre en oeuvre, les dispositifs d'injection de substance 6 sont des conduites d'injection de substance 6, essentiellement parallèles entre elles et, de préférence, orthogonales aux plaques d'échange thermique 1.

Les conduites d'injection de substance 6 peuvent par exemple être fabriquées en acier inoxydable ou en céramique poreuse.

Les conduites d'injection de substance 6 traversent les plaques d'échange thermique 1 au niveau des zones de jonction 5 décrites ci-dessus. Ainsi, on évite tout risque de communication entre les espaces d'échange thermique 4 occupés par le fluide caloporteur et les compartiments réactionnels 3 occupés par le flux réactionnel.

Pour ce faire, les zones de jonction 5 doivent présenter des dimensions suffisantes pour permettre le percement d'une ouverture 7 au sein de ces zones de jonction 5 sans compromettre l'étanchéité de l'espace d'échange thermique 4, l'ouverture 7 étant adaptée au passage de la conduite d'injection de substance 6.

Les conduites d'injection de substance 6 ont par exemple un diamètre de 10 à 100 mm, et de préférence de 20 à 50 mm (afin qu'elles aient une rigidité suffisante pour permettre l'assemblage). Les zones de jonction 5, qui sont par exemple constituées de soudures de forme circulairerprésentent de préférence en leur centre des ouvertures 7 respectifs de diamètre légèrement supérieur (par exemple de 1 à 5 mm) à celui des conduites d'injection de substance 6.

Des moyens de scellement ou de jointoiement peuvent être prévus autour des conduites d'injection de substance 6 au niveau des ouvertures 7 sur les zones de jonction 5 si l'on souhaite éviter toute communication entre compartiments réactionnels 3.

Par ailleurs, chaque conduite d'injection de substance 6 comporte des moyens d'injection de substance, qui peuvent être de simples orifices, de préférence sur l'ensemble de sa longueur traversant les compartiments réactionnels 3.

L'ensemble des conduites d'injection de substance 6 peuvent être reliées à au moins un système de distribution de substance 8 (lui-même connecté à une réserve de substance à injecter). On peut prévoir des systèmes de distribution de substance indépendants entre les modules ou un système de distribution de substance unique pour l'ensemble des modules. Eventuellement, on prévoit également au moins un système de collecte de substance (non représenté) ou plusieurs, par exemple un par module, à l'extrémité des conduites d'injection de substance 6 opposée par rapport au(x) système(s) de distribution de substance 8. Cela s'avère utile lorsque l'on souhaite qu'une partie seulement du flux de substance circulant dans la conduite 6 passe dans les compartiments réactionnels 3 traversés (par exemple afin de garantir une pression suffisante en tout point d'injection).

Lorsque les zones de jonction 5 sont sous forme de points, on peut prévoir un dispositif d'injection de substance 6 traversant chaque zone de jonction 5 ; ou bien on peut prévoir des dispositifs d'injection de substance 6 traversant seulement certaines zones de jonction 5, les autres zones de jonction 5 étant alors de préférence dépourvues d'ouverture 7 : c'est ce mode de réalisation qui est représenté sur les **figures 1** à **4****.**

Lorsque les zones de jonction 5 sont sous forme de bandes, on peut prévoir un dispositif d'injection de substance 6 traversant chaque bande ; ou bien on peut prévoir des dispositifs d'injection de substance 6 traversant seulement certaines bandes, les autres bandes étant alors de préférence dépourvues d'ouverture 7. On peut également prévoir plusieurs dispositifs d'injection de substance 6 traversant une même bande, celle-ci étant percée d'un nombre d'ouvertures adapté au nombre de dispositifs d'injection de substance 6 la traversant.

De préférence, la disposition des dispositifs d'injection de substance 6 permet une injection essentiellement homogène de la substance dans les compartiments réactionnels 3.

La substance injectée dans les compartiments réactionnels 3 au moyen des dispositifs d'injection de substance peut être un gaz ou un liquide ou un mélange des deux.

Il peut s'agir d'un réactif ou d'un composé catalytique ou d'un composé apte à régénérer le catalyseur présent dans les compartiments réactionnels 3.

Il est possible de procéder à l'injection de substance dans les compartiments réactionnels 3 uniquement au cours de la phase de production, ou uniquement au cours de la phase de régénération, ou au cours des deux phases.

Il est également possible d'injecter des substances différentes au cours du temps (par exemple une première substance lorsque le réacteur fonctionne en phase de production, et une deuxième substance lorsque le réacteur fonctionne en phase de régénération).

De préférence, la substance injectée est un gaz comprenant de l'oxygène, par exemple de l'oxygène quasi-pur, ou de l'air, ou un mélange de gaz inerte et d'oxygène.

En effet, pour les réactions dans lesquelles le catalyseur se désactive par cokage, l'injection d'oxygène au cours de la phase de régénération permet d'effectuer une combustion et donc une régénération du catalyseur, et ce avec un meilleur contrôle de la température dans l'ensemble des compartiments réactionnels et avec une plus grande sécurité. En particulier, on peut ainsi éviter un déplacement de front chaud à travers tout le lit de catalyseur. Et l'injection d'oxygène au cours de la phase de production peut permettre d'allonger la durée de désactivation du catalyseur, avec encore les bénéfices susmentionnés en termes de contrôle de la température et de sécurité améliorée.

L'injection d'oxygène est également utile pour les catalyseurs dont la régénération implique une oxygénation, comme par exemple les catalyseurs de type molybdate de bismuth utilisés pour l'oxydation du propylène en acroléine et de l'isobutène / t-butanol en méthacroléine, ou les catalyseurs de type molybdate de fer utilisés pour de multiples réactions comme l'oxydation du méthanol en formaldéhyde, l'oxydation de l'éthanol en acétaldéhyde, l'oxydation du méthanol en diméthoxyméthane, l'oxydation de l'éthanol en diéthoxyéthane...

Un autre exemple de substance injectée est le chlore, en particulier dans le cadre de la mise en oeuvre de réactions de fluoration. En effet l'injection de chlore en phase de production permet d'inhiber des réactions de désactivation de catalyseur.

Si besoin, le flux de la substance injectée peut être ajusté de manière différente selon les emplacements d'injection. Des moyens de pompage ou de compression et des vannes permettent de régler le débit de substance dans chaque dispositif d'injection de substance 6 et dans chaque compartiment réactionnel 3.

### Réactions pouvant être mise en oeuvre selon l'invention

L'invention est avantageusement mise en oeuvre pour la production d'acide acrylique d'origine renouvelable, en particulier la production d'acide acrylique à partir de glycérol, comportant une première étape de déshydratation du glycérol en acroléine suivie d'une étape d'oxydation en phase gaz de l'acroléine ainsi obtenue ; ou dans la production d'acide acrylique par déshydratation des acides 2-hydroxypropionique (acide lactique) ou 3-hydroxypropionique et de leurs esters.

L'invention permet notamment de mettre en oeuvre un procédé de déshydratation du glycérol en acroléine. Dans ce type de procédé, le glycérol est alimenté sous forme d'une solution aqueuse avec une concentration de 20 à 95 % en masse. Plus le glycérol est concentré et plus les catalyseurs ont tendance à former du coke, ce qui nécessite de régénérer le catalyseur régulièrement. Selon le procédé décrit dans la demande WO 2006/087083, la réaction est avantageusement mise en oeuvre en présence d'oxygène, l'ajout d'oxygène dans la réaction de déshydratation de glycérol permettant de prolonger la durée de vie du catalyseur et d'espacer les régénérations.

La réaction est typiquement effectuée à une température de 220 à 350°C et de préférence de 280 à 320°C ; et à une pression variant de la pression atmosphérique à quelques bars (par exemple 5). La concentration des réactifs est en partie dictée ou imposée par les limites d'inflammabilité. Dans les réacteurs de l'art antérieur, les limites d'inflammabilité d'un mélange gazeux O₂ / gaz inerte / glycérol / vapeur d'eau imposent une faible concentration en glycérol et oxygène en phase gazeuse.

Avec le réacteur de l'invention, il est possible de mettre en oeuvre un mélange plus riche en glycérol qu'avec les réacteurs de l'art antérieur, car un complément d'oxygène est apporté par injection *in situ.* Par exemple on peut utiliser des mélanges contenant 6 % de glycérol, et 1 à 2 % d'oxygène, en apportant le complément *in situ.*

Les catalyseurs utilisables pour cette réaction sont des catalyseurs acides, ayant notamment une acidité de Hammett inférieure à +2, tels que décrits par exemple dans les documents EP 1 848 681, WO 2009/12855, WO 2009/044081 ou WO 2010/046227 De nombreux catalyseurs acides peuvent convenir pour cette réaction. On citera les zircones phosphatées, les zircones tungstées, les zircones silicées, les oxydes de titane ou d'étain imprégnés de tungstate ou phosphotungstate ou silicotungstate, les alumines ou silices phosphatées, les hétéropolyacides ou sels d'hétéropolyacides, les phosphates de fer et les phosphates de fer comprenant un promoteur.

Dans le cadre de ce procédé, le catalyseur subit une désactivation notamment par cokage. Cette désactivation peut toutefois être retardée par l'injection d'oxygène *in situ* dans les compartiments réactionnels du réacteur, en phase de production. Le rapport molaire O₂ / glycérol en entrée de réacteur est de 0,1 à 1,5 (de préférence de 0,3 à 1,0) et la pression partielle en oxygène est inférieure à 7 %. L'ajout d'oxygène complémentaire sous forme d'oxygène moléculaire, d'air ou de mélange d'oxygène et d'un gaz inerte pour la réaction est réparti sur la longueur du réacteur. De préférence, on prévoit entre 2 et 10 sites d'injection sur la longueur du réacteur. La pression partielle cumulée de l'oxygène ajouté le long du réacteur est ainsi de 1 à 10 %.

Par ailleurs, en phase de régénération, l'injection d'oxygène *in situ* dans les compartiments réactionnels du réacteur permet de régénérer le catalyseur de manière sûre et contrôlée.

De manière générale en phase de régénération, il est nécessaire de bien contrôler la température du catalyseur pour éviter des conditions d'emballement de la réaction, conduisant à des élévations très importantes de la température du catalyseur pouvant l'endommager irréversiblement et même endommager le réacteur. On cherche donc à limiter la température que peut atteindre le catalyseur en diluant très fortement le flux gazeux et / ou en ajoutant des composés inertes caloporteurs comme de la vapeur d'eau ou du CO₂. La régénération prend alors beaucoup de temps car un front chaud se déplace à travers le lit de catalyseur correspondant à la combustion du coke. II est alors souhaitable d'accélérer la réaction de régénération par combustion du coke. Cette régénération peut être accélérée en augmentant la température et ou la pression partielle d'oxygène, mais on risque alors de rencontrer les inconvénients énumérés ci-dessus. Le réacteur de l'invention permet de répartir l'ajout d'oxygène en plusieurs points du réacteur, et par conséquent d'effectuer une régénération en multipliant les fronts chauds dans le réacteur. Il est donc souhaitable d'injecter l'oxygène en de multiples lignes et de préférence avec des lignes réparties tous les 5 à 33 % de la longueur du réacteur, et de préférence de 10 à 20 % de la longueur du réacteur.

L'invention permet également de mettre en oeuvre un procédé de déshydratation de l'acide lactique ou de l'acide 3-hydroxypropionique (et de leurs esters correspondants) en acide acrylique.

L'acide lactique a un point d'ébullition voisin de 217 °C et l'acide 3-Hydroxypropionique a un point d'ébullition de 279 °C (valeur calculée). Les limites d'inflammabilité pour l'acide lactique dans l'air sont de 3,1 % (Limite inférieure) et de 18 % (limite supérieure). L'ester méthylique de l'acide lactique a un point d'ébullition de 145 °C, pour les limites d'inflammabilité de 1,1 et 3,6 % (ce qui donne une plus grande flexibilité d'utilisation que pour l'acide). L'ester méthylique de l'acide 3-hydroxypropionique a un point d'ébullition de 179 °C (180 °C en valeur calculée), L'ester éthylique de l'acide lactique a un point d'ébullition de 154 °C, et des limites d'inflammabilité de 1,6 et 10,6 %. L'ester éthylique de l'acide 3-hydroxypropionique a un point d'ébullition de 187,5 °C.

Pour ces réactions, on utilise une configuration de réacteur sensiblement identique à celle de la déshydratation du glycérol. Les conditions de déshydratation sont une température de 220 à 400°C, et de préférence de 250 à 350°C, et une pression de 0,5 à 5 bar.

Les catalyseurs qui peuvent convenir à ces réactions sont des catalyseurs acides, ayant notamment une acidité de Hammett inférieure à +2. Les catalyseurs peuvent être choisis parmi des matériaux siliceux naturels ou de synthèse ou les zéolithes acides ; des supports minéraux, tels que des oxydes, recouverts par des acides inorganiques, mono, di, tri ou polyacides ; des oxydes ou oxydes mixtes ou encore des hétéropolyacides ou sels d'hétéropolyacides contenant notamment au moins un élément choisi parmi le groupe comprenant W, Mo et V. Parmi les oxydes mixtes, on peut citer particulièrement ceux à base de fer et de phosphore et ceux à base de césium, phosphore et tungstène.

D'autres catalyseurs qui peuvent aussi convenir pour ces réactions sont obtenus à partir de phosphates et/ou de sulfates des métaux alcalins, alcalino-terreux et des terres rares, et leurs mélanges. Dans ce groupe on trouve ainsi les phosphates et oxyphosphates de lanthane, les phosphates de sodium, les phosphates de calcium, le sulfate de calcium, le sulfate de magnésium, et les hydrogénophosphates correspondants. Le phosphate d'aluminium, le phosphate de bore. Toutes les matières actives citées ci-dessus peuvent être imprégnées ou enrobées sur tout type de support tel que : alumine, oxyde de titane, oxyde de zirconium, silice, mais aussi les oxydes mixtes correspondants, et le carbure de silicium.

La pression partielle en acide lactique ou 3-hydroxypropionique est généralement de 1 à 10 % et de préférence de 2 à 6 %.

Dans le cadre de ces réactions également, le catalyseur subit une désactivation notamment par cokage. Cette désactivation peut être retardée en injectant de l'oxygène *in situ* dans les compartiments réactionnels du réacteur, en phase de production. Les conditions relatives à l'injection d'oxygène sont les mêmes que celles décrites ci-dessus en relation avec la déshydratation du glycérol.

Par ailleurs, en phase de régénération, l'injection d'oxygène *in situ* dans les compartiments réactionnels du réacteur permet de régénérer le catalyseur de manière sûre et contrôlée. Les conditions relatives à l'injection d'oxygène sont les mêmes que celles décrites ci-dessus en relation avec la déshydratation du glycérol.

L'invention permet également de mettre en oeuvre un procédé de déshydratation de l'acide 2-hydroxyisobutyrique ou de l'acide 3-hydroxyisobutyrique en acide méthacrylique.

Pour ce type de réaction, on utilise une configuration de réacteur sensiblement identique à celle de la déshydratation du glycérol. Les conditions de déshydratation sont une température de 200 à 400°C, et de préférence de 250 à 350°C, et une pression de 0,5 à 5 bar. Les catalyseurs qui peuvent convenir à cette réaction sont des catalyseurs acides, ayant notamment une acidité de Hammett inférieure à +2. Les catalyseurs peuvent être choisis parmi des matériaux siliceux naturels ou de synthèse ou les zéolithes acides ; des supports minéraux, tels que des oxydes, recouverts par des acides inorganiques, mono, di, tri ou polyacides ; des oxydes ou oxydes mixtes ou encore des hétéropolyacides ou sels d'hétéropolyacides contenant notamment au moins un élément choisi parmi le groupe comprenant W, Mo et V. Parmi les oxydes mixtes, on peut citer particulièrement ceux à base de fer et de phosphore et ceux à base de césium, phosphore et tungstène.

Des catalyseurs qui peuvent aussi convenir pour cette réaction sont obtenus à partir de phosphates et/ou de sulfates des métaux alcalins, alcalino-terreux et des terres rares, et leurs mélanges. Dans ce groupe on trouve ainsi les phosphates et oxyphosphates de lanthane, les phosphates de sodium, les phosphates de calcium, le sulfate de calcium, le sulfate de magnésium, et les hydrogénophosphates correspondants. Le phosphate d'aluminium, le phosphate de bore. Toutes les matières actives citées ci-dessus peuvent être imprégnées ou enrobées sur tout type de support tel que : alumine, oxyde de titane, oxyde de zirconium, silice, mais aussi les oxydes mixtes correspondants, et le carbure de silicium.

La pression partielle en acide (2- ou 3-)hydroxyisobutyrique est généralement de 1 à 10 % et de préférence de 2 à 6 %.

Dans le cadre de ces réactions également, le catalyseur subit une désactivation notamment par cokage. Cette désactivation peut être retardée en injectant de l'oxygène *in situ* dans les compartiments réactionnels du réacteur, en phase de production. Les conditions relatives à l'injection d'oxygène sont les mêmes que celles décrites ci-dessus en relation avec la déshydratation du glycérol.

Par ailleurs, en phase de régénération, l'injection d'oxygène *in situ* dans les compartiments réactionnels du réacteur permet de régénérer le catalyseur de manière sûre et contrôlée. Les conditions relatives à l'injection d'oxygène sont les mêmes que celles décrites ci-dessus en relation avec la déshydratation du glycérol.

L'invention permet également de mettre en oeuvre des oxydations sélectives telles que l'oxydation du méthanol en formaldéhyde ou en diméthoxyméthane ; l'oxydation de l'éthanol en acétaldéhyde ou en diethoxyéthane ; l'oxydation de l'orthoxylène ou du naphtalène en anhydride phtalique, l'oxydation du benzène, du butène, du butanol ou du butane en anhydride maléique.

Dans ces réactions d'oxydation la problématique commune est d'injecter en entrée de réacteur une quantité suffisante d'oxygène (ou autre agent oxydant) pour assurer une conversion totale, ou du moins la plus élevée possible, du réactif hydrocarboné, en s'assurant de limiter les risques d'emballement du réacteur et les risques d'explosion. La solution retenue revient à limiter la productivité des réacteurs, en limitant la pression partielle du composé hydrocarboné. L'invention permet d'utiliser des ratios O₂ / réactif hydrocarboné plus faibles en entrée de réacteur, le reste de l'oxygène nécessaire à la réaction étant apporté par les dispositifs d'injection.

Dans le cas des réactions d'oxydation du méthanol en formaldéhyde ou diméthoxyméthane, et d'oxydation de l'éthanol en acétaldéhyde ou diéthoxydéthane, les catalyseurs qui peuvent convenir sont des oxydes mixtes, tels que les oxydes de molybdène et de fer, les oxydes de molybdène contenant des métaux choisis parmi le bismuth, le vanadium, le tungstène, le cuivre, le nickel, le cobalt. Les conditions opératoires sont une température comprise entre 200 et 350°C, de préférence entre 250 et 300°C; une pression comprise entre 1 et 5 bar. La pression partielle en alcool peut varier dans une large gamme de 3 à 50 %, et de préférence de 5 à 40 %, suivant le type de produit recherché. Dans le cas où les aldéhydes sont les produits recherchés, la pression partielle en alcool est comprise entre 3 et 10 % et de préférence entre 5 et 9 %. Dans le cas où les acétals sont les produits recherchés, la pression partielle en alcool est comprise entre 10 et 50 % et de préférence entre 20 et 40 %.

Dans le cas des réactions d'oxydation de l'orthoxylène et du naphtalène en anhydride phtalique, les catalyseurs sélectionnés contiennent de préférence du vanadium, et de préférence de l'oxyde de vanadium supporté. Les conditions opératoires sont une pression de 1 à 5 bar, et des températures de réaction de 280 à 450°C.

Dans le cas des réactions d'oxydation du butane, des butènes, du butanol et du benzène en anhydride maléique, les catalyseurs qui conviennent contiennent du vanadium, sous forme d'oxyde de vanadium supporté ou sous forme de d'oxyde mixte vanadium-phosphore supporté. Les températures de réactions sont de 350 à 500°C, et les pressions de 1 à 5 bar.

Dans le cas des réactions d'oxydation du propylène en acroléine, de l'isobutène ou du tertiobutanol en méthacroléine, les catalyseurs qui conviennent sont constitués majoritairement de molybdène, et contiennent des éléments choisis parmi (mais pas exclusivement) les éléments suivants : nickel, fer, cobalt, tungstène, potassium, bismuth, antimoine, chrome. Les températures de réaction sont comprises entre 320 et 450 °C. Les pressions totales sont comprises entre 1 et 5 bar. Les pressions partielles en composé hydrocarboné sont comprises entre 5 et 15 %, et le rapport O₂ / composé hydrocarboné en entrée de réacteur est compris entre 0,5 et 4 et de préférence entre 0,8 et 2, et encore plus de préférence entre 1 et 1,8, et encore plus préféré entre 1,2 et 1,6.

Dans le cas des réactions d'oxydation de l'acroléine en acide acrylique, et de la méthacroléine en acide méthacrylique, les catalyseurs qui conviennent sont constitués majoritairement de molybdène, et contiennent des éléments choisis parmi les éléments suivants (mais pas exclusivement) : vanadium, tungstène, cuivre, antimoine, niobium, strontium, phosphore, fer. Les températures de fonctionnement sont comprises entre 250 et 350 °C, pour une pression totale de 1 à 5 bar. La pression partielle en aldéhyde est comprise entre 5 et 15 %, et le ratio O₂ / aldéhyde en entrée de réacteur est compris entre 0,3 et 1,2, et de préférence compris entre 0,5 et 1.

D'autres réactions d'oxydation pouvant être mises en oeuvre selon l'invention sont :
- La production d'acide acrylique à partir de propylène et d'oxygène, les coproduits étant l'acroléine, l'acide acétique, l'acide maléique, l'acide propionique, l'acétaldéhyde et l'acétone, par exemple à une température de 300 à 400°C et à une pression de 1 à 3 bar.
- La production d'oxyde d'éthylène à partir d'éthylène et d'oxygène, les coproduits étant l'acétaldéhyde et le formaldéhyde, par exemple à une température de 230 à 290°C et à une pression de 10 à 30 bar.
- La production de 1,2-dichloroéthane à partir d'éthylène, d'acide chlorhydrique et d'oxygène, les coproduits étant le monoxyde de carbone, le chloral et divers composés chlorés, par exemple à une température de 220 à 300°C et à une pression de 2 à 6 bar.
- La production d'acide téréphtalique à partir de p-xylène et d'oxygène, les coproduits étant l'anhydride maléique, l'acide o-toluique et l'acide benzoïque, par exemple à une température de 175 à 230°C et à une pression de 15 à 30 bar.

Le réacteur selon l'invention peut aussi convenir pour des réactions d'ammoxydation mettant en jeu des mélanges ammoniac-oxygène-inerte-composé hydrocarboné. Les composés hydrocarbonés qui peuvent être utilisés comprennent le propylène, l'isobutène, l'acroléine, la méthacroléine, mais aussi des composés aromatiques. Les réactions d'ammoxydation sont effectuées à une température de 50 à 100 °C plus élevée que les températures d'oxydation correspondantes. Le réacteur selon l'invention permet d'injecter l'un ou l'autre réactif par les dispositifs d'injection. Eventuellement on peut alterner les réactifs injectés selon la longueur du réacteur. De préférence, l'oxygène (ou l'air / air enrichi) est injecté par les dispositifs d'injection. Néanmoins dans le cas des réactions d'ammoxydation d'une aldéhyde telle que l'acroléine, l'injection multiétagée de l'ammoniac par les dispositifs d'injection permet de limiter des réactions parasites qui tendent à former des dépôts en entrée de réacteur.

A titre d'exemple, on peut produire l'acrylonitrile (en coproduisant de l'acétonitrile, de l'acide hydrocyanique et du monoxyde de carbone) à partir de propylène et / ou propane, d'oxygène et d'ammoniac, par exemple à une température de 400 à 500°C et à une pression de 1 à 4 bar.

L'invention permet également de mettre en oeuvre un procédé de fluoration, c'est-à-dire un procédé de préparation d'un composé fluoré à partir d'un composé chloré. De préférence; la réaction est effectuée en faisant réagir le composé chloré avec du fluorure d'hydrogène (HF).

Le composé chloré peut être toute molécule possédant au moins un atome de chlore et le composé fluoré peut être toute molécule possédant au moins un atome de fluor.

De préférence, le composé chloré est un alcane ou un alcène en C2, ou en C3, ou en C4, ou en C5, linéaire ou ramifié (de préférence linéaire), comportant un ou plusieurs substituants choisis parmi F, Cl, I et Br (de préférence parmi F et Cl), au moins un des substituants étant Cl.

De préférence, le composé fluoré est un alcane ou un alcène en C2, ou en C3, ou en C4, ou en C5, linéaire ou ramifié (de préférence linéaire), comportant un ou plusieurs substituants choisis parmi F, Cl, I et Br (de préférence parmi F et Cl), au moins un des substituants étant F.

De manière plus particulièrement préférée, le composé chloré est un alcane ou un alcène en C3 comportant un ou plusieurs substituants choisis parmi F, Cl, I et Br (de préférence parmi F et Cl), au moins un des substituants étant Cl ; et le composé fluoré est un alcène en C3 comportant un ou plusieurs substituants choisis parmi F, Cl, I et Br (de préférence parmi F et Cl), au moins un des substituants étant F.

Alternativement, le composé chloré peut être un alcane ou un alcène en C4 comportant un ou plusieurs substituants choisis parmi F, Cl, I et Br (de préférence parmi F et Cl), au moins un des substituants étant Cl ; et le composé fluoré est un alcène en C4 comportant un ou plusieurs substituants choisis parmi F, Cl, I et Br (de préférence parmi F et Cl), au moins un des substituants étant F.

Selon un mode de réalisation, le composé fluoré est une hydrofluorooléfine (et ne comporte donc pas de substituant Cl).

De préférence, au cours de la réaction, au moins un substituant Cl du composé chloré est remplacé par un substituant F.

La conversion du composé chloré en le composé fluoré comprend la conversion directe (en une seule étape ou selon un seul ensemble de conditions opératoires) et la conversion indirecte (en deux ou plus de deux étapes ou en utilisant plus d'un ensemble de conditions opératoires).

Les réactions de fluoration plus particulièrement préférées sont les réactions :
- du 2-chloro-3,3,3-trifluoro-1-propène (HFCO-1233xf) en 2,3,3,3-tétrafluoro-1-propène (HFO-1234yf) ;
- du 1,1,1,2,3-pentachloropropane (HCC-240db) en 2,3,3,3-tétrafluoro-1-propène (HFO-1234yf) ;
- du 1,1,2,2,3-pentachloropropane (HCC-240aa) en 2,3,3,3-tétrafluoro-1-propène (HFO-1234yf) ;
- du 2,3-dichloro-1,1,1-trifluoropropane (HCFC-243db) en 2,3,3,3-tétrafluoro-1-propène (HFO-1234yf) ;
- du 1,1,2,3-tétrachloro-1-propène (HCO-1230xa) en 2,3,3,3-tétrafluoro-1-propène (HFO-1234yf) ;
- du 2,3,3,3-tétrachloro-1-propène (HCO-1230xf) en 2,3,3,3-tétrafluoro-1-propène (HFO-1234yf) ;
- du 1,1,1,2,3-pentachloropropane (HCC-240db) en 2-chloro-3,3,3-trifluoro-1-propène (HFCO-1233xf) ;
- du 1,1,2,2,3-pentachloropropane (HCC-240aa) en 2-chloro-3,3,3-trifluoro-1-propène (HFCO-1233xf) ;
- du 2,3-dichloro-1,1,1-trifluoropropane (HCFC-243db) en 2-chloro-3,3,3-trifluoro-1-propène (HFCO-1233xf) ;
- du 1,1,2,3 tétrachloro-1-propène (HCO-1230xa) en 2-chloro-3,3,3-trifluoro-1-propène (HFCO-1233xf) ;
- du 2,3,3,3 tétrachloro-1-propène (HCO-1230xf) en 2-chloro-3,3,3-trifluoro-1-propène (HFCO-1233xf).

La réaction de fluoration peut être effectuée avec un rapport molaire de HF typiquement de 3:1 à 150:1, avec un temps de contact de 6 à 100 s et à une pression de la pression atmosphérique à 20 bar. La température de la réaction peut être de 200 à 450°C.

Un exemple particulier de procédé permettant de préparer du HFO-1234yf à partir de HFCO-1233xf figure dans le document WO 2010/123154. Ce procédé peut être mis en oeuvre avec le réacteur selon l'invention.

Le catalyseur utilisé pour les réactions de fluoration ci-dessus peut être supporté ou non.

Il peut s'agir par exemple d'un catalyseur métallique, c'est à dire du type métal élémentaire, oxyde métallique, halogénure métallique et / ou sel métallique), en particulier un oxyde de métal de transition ou un halogénure ou un oxyhalogénure d'un tel métal.

Il peut s'agir notamment d'halogénure d'antimoine, d'halogénure d'étain, d'halogénure de thallium, d'halogénure de thallium, d'halogénure de fer et de combinaison de ceux-ci. Les chlorures et fluorures métalliques sont préférés, par exemple SbCl₅, SbCl₃, SbF₅, SnCl₄, TiCl₄, FeCl₃ et les combinaisons de ceux-ci.

D'autres catalyseurs appropriés sont ceux à base de chrome, tels que l'oxyfluorure de chrome, les oxydes de chrome tels que Cr₂O₃ (éventuellement soumis à des traitements de fluoration), les fluorures de chrome et les combinaisons de ceux-ci.

D'autres catalyseurs possibles sont ceux à base d'aluminium (par exemple AlF₃, Al₂O₃ et oxyfluorure d'aluminium).

Le catalyseur peut être choisi parmi l'alumine fluorée, le dioxyde de titane fluoré, l'acier inoxydable fluoré, le charbon actif et le graphite.

Il peut s'agir d'un mélange de chrome et de magnésium (sous forme élémentaire, saline, oxyde, ou halogénure) ; ou d'un mélange de chrome et d'un autre métal (sous forme élémentaire, saline, oxyde, ou halogénure).

Il peut également s'agir d'un catalyseur comprenant un métal sur un support.

Le métal peut être choisi parmi les groupes 3, 4, 5, 6, 7, 8, 9 et 13 du tableau périodique, et peut notamment être Al, Cr, Mn, Co, Ni, Zn, Ti, V, Ru, Rh, Pd, Os, Ir, Pt, Zr, Mo, Re, Sc, Y, La, Hf, Cu, Ag, Au, Ge, Sn, Pb ou Mg, notamment Al, Cr, Mn, Ni ou Co. Il peut s'agir d'un lanthanide (métaux 58 à 71 du tableau périodique). Le métal du catalyseur peut être converti en dérivés métalliques lors de son activation ou de sa régénération, par exemple en oxyde, halogénure (fluorure, bromure, chlorure), oxyhalogénure ou pseudohalogénure (cyanure, cyanate, thiocyanate).

Le support peut être choisi parmi l'aluminium, les halogénures d'aluminium et les oxyhalogénures d'aluminium, l'alumine, l'alumine activée, l'alumine fluorée, le fluorure d'aluminium, le charbon actif (fluoré ou non) et le graphite (éventuellement fluoré).

Le catalyseur peut être préparé par exemple par immersion du support dans une solution de composé soluble (par exemple nitrate ou chlorure) du métal, ou alternativement la solution peut être pulvérisée sur le support. Le support peut être séché et mis en contact avec un agent d'halogénation sous forme vapeur (par exemple fluorure d'hydrogène, acide chlorhydrique, chlorofluorohydrocarbure, ou encore SiF₄, CCl₃F, CCl₂F, CHF₃, CCl₂FCClF₂) avec chauffage afin d'halogéner partiellement ou complètement le support ou le métal.

Des exemples de catalyseurs supportés sont FeCl₃ supporté sur du carbone, l'alumine supportée sur du carbone, le fluorure d'aluminium supporté sur du carbone, l'alumine fluorée supportée sur du carbone, le fluorure de magnésium supporté sur du fluorure d'aluminium, plus généralement les métaux (métaux élémentaires, oxydes métalliques, halogénures métalliques et / ou sels métalliques) supportés sur du fluorure d'aluminium, les métaux supportés sur de l'alumine, les métaux supportés sur du carbone, les mélanges de métaux.

D'autres exemples de catalyseurs supportés sont : un halogénure de magnésium ou un halogénure de zinc supporté sur Cr₂O₃, un halogénure de chrome III supporté sur du carbone, un mélange de chrome et magnésium (sous forme élémentaire, oxyde, halogénure ou saline) supporté sur du graphite, un mélange de chrome et d'un autre métal (sous forme élémentaire, saline, oxyde, ou halogénure) supporté sur du graphite ou de l'alumine ou un halogénure d'aluminium tel que le fluorure d'aluminium.

La teneur métallique totale du catalyseur supporté est de préférence de 0,1 à 20 % en masse, par exemple de 0,1 à 10 % (par rapport à la masse totale du catalyseur).

Un mode de réalisation préféré utilise un catalyseur particulier, qui est un catalyseur mixte supporté, contenant à la fois du chrome et du nickel. Le rapport molaire Cr:Ni (par rapport aux éléments métalliques) est généralement de 0,5 à 5, par exemple de 0,7 à 2, notamment voisin de 1. Le catalyseur peut contenir de 0,5 à 20 % de chrome et de 0,5 à 20 % de nickel en masse, de préférence de 2 à 10 % de chaque métal. On peut faire référence à cet égard au document WO 2009/118628, et en particulier à la description du catalyseur de la p.4, I.30 à la p.7, I.16.

Un catalyseur intéressant est un catalyseur à base de chrome comprenant un composé chromé du type oxyde de chrome cristallin sous forme alpha, dans lequel de 0,05 à 6 % des atomes environ du réseau d'oxyde de chrome alpha sont remplacés par des atomes de cobalt trivalents (éventuellement traité par un agent de fluoration). On renvoie au document US 2005/0228202 à ce sujet.

Le catalyseur peut subir une fluoration. A titre d'exemple de fluoration, on peut préparer une alumine fluorée en mettant en contact une alumine avec du fluorure d'hydrogène avec chauffage, ou en pulvérisant une solution aqueuse de fluorure d'hydrogène à température ambiante ou en immergeant une alumine en solution, puis en séchant. La fluoration du catalyseur peut être mise en oeuvre dans le réacteur selon l'invention ou non. La température lors de la fluoration est généralement de 200 à 450°C.

Le catalyseur peut éventuellement contenir une faible teneur d'un ou plusieurs co-catalyseurs tels que des sels de Co, Zn, Mn, Mg, V, Mo, Te, Nb, Sb, Ta, P et Ni. Un co-catalyseur préféré est le nickel. Un autre co-catalyseur préféré est le magnésium.

Par exemple, un catalyseur à base de chrome non supporté peut éventuellement contenir une faible teneur d'un ou plusieurs co-catalyseurs choisis parmi le cobalt, le nickel, le zinc ou le manganèse, et être préparé par des procédés connus en soi, tels que l'imprégnation, le mélange de poudres et autres.

La quantité de co-catalyseur, lorsqu'il est présent, peut varier de 1 à 10 % en masse, de préférence de 1 à 5 % en masse. Le co-catalyseur peut être ajouté au catalyseur par des procédés connus en soi tels que l'adsorption à partir d'une solution aqueuse ou organique, suivie de l'évaporation du solvant. Un catalyseur préféré est de l'oxyde de chrome pur avec du nickel ou du zinc en tant que co-catalyseur. Alternativement, le co-catalyseur peut être physiquement mélangé avec le catalyseur par broyage pour produire un mélange fin.

Un autre catalyseur préféré est un catalyseur mixte chrome / nickel supporté sur de l'alumine fluorée. Le document US 5,731,481 décrit un procédé de préparation de cet autre catalyseur.

Avant activation, le catalyseur est soumis à une étape de séchage, de préférence avec un gaz de séchage tel que l'azote. L'étape de séchage peut être effectuée à une pression allant de la pression atmosphérique à 20 bar. La température du catalyseur lors de l'étape de séchage peut aller de 20°C à 400°C, de préférence de 100 à 200°C.

L'activation du catalyseur peut être de préférence effectuée avec HF ou un fluoro ou hydrofluoroalcane et / ou un agent oxydant (oxygène ou chlore de préférence). La régénération du catalyseur peut être effectuée avec un agent oxydant (oxygène ou chlore de préférence) et éventuellement HF, en une ou plusieurs étapes.

Afin d'allonger la durée de désactivation du catalyseur, il est possible d'ajouter un agent oxydant (oxygène ou chlore de préférence) lors de la phase de production, par exemple à raison de 0,05 à 15 mol% par rapport au mélange d'agent oxydant et de composé chloré.

La température lors de l'étape de régénération peut être de 250 à 500°C environ et la pression de la pression atmosphérique à environ 20 bar. Lorsqu'on utilise HF en combinaison avec de l'oxygène, la proportion d'oxygène peut varier de 2 à 98 mol% par rapport au mélange HF / oxygène.

L'invention permet d'effectuer notamment l'injection d'oxygène (ou de chlore) *in situ* dans le réacteur en phase de production et / ou en phase de régénération, ainsi qu'éventuellement pour l'activation initiale du catalyseur.

## Revendications

1. Réacteur chimique comprenant une pluralité de plaques d'échange thermique (1) définissant entre elles des compartiments réactionnels (3), dans lequel chaque plaque d'échange thermique (1) comporte deux parois (2a, 2b) définissant entre elles au moins un espace d'échange thermique (4), les parois (2a, 2b) respectives étant fixées entre elles par des zones de jonction (5), et le réacteur comprend également au moins un dispositif d'injection de substance (6) dans les compartiments réactionnels (3), ledit dispositif d'injection de substance (6) traversant les plaques d'échange thermique (1) dans des zones de jonction (5) respectives de celles-ci.

2. Réacteur chimique selon la revendication 1, dans lequel le dispositif d'injection de substance (6) est un dispositif d'injection de gaz, et de préférence un dispositif d'injection de gaz comprenant de l'oxygène.

3. Réacteur chimique selon la revendication 1 ou 2, dans lequel le dispositif d'injection de substance (6) est une conduite présentant une pluralité d'orifices d'injection.

4. Réacteur chimique selon l'une des revendications 1 à 3, dans lequel les zones de jonction (5) sont disposées sur les plaques d'échange thermique (1) sous forme de bandes, de préférence parallèles, ou sous forme de points.

5. Réacteur chimique selon l'une des revendications 1 à 4, comportant une pluralité de dispositifs d'injection de substance (6), de préférence parallèles entre eux, et de préférence connectés à un système de distribution de substance (8).

6. Réacteur chimique selon l'une des revendications 1 à 5, dans lequel les plaques d'échange thermique (1) sont disposées dans une enceinte de manière radiale, ou de manière parallèle entre elles, et de préférence sont groupées en modules.

7. Procédé de réaction chimique, comprenant l'amenée de réactifs en entrée d'un compartiment réactionnel (3) défini entre deux plaques d'échange thermique (1), le soutirage de produits de réaction en sortie du compartiment réactionnel (3), ainsi que l'injection d'une substance dans le compartiment réactionnel (3), dans lequel chaque plaque d'échange thermique (1) comporte deux parois (2a, 2b) définissant entre elles au moins un espace d'échange thermique (4), les parois (2a, 2b) respectives étant fixées entre elles par des zones de jonction (5), et l'injection de substance étant effectuée au moyen d'au moins un dispositif d'injection de substance (6) traversant les plaques d'échange thermique (2) dans des zones de jonction (5) respectives de celles-ci.

8. Procédé selon la revendication 7, dans lequel la réaction est du type catalytique hétérogène et dans lequel un catalyseur est disposé dans les compartiments réactionnels (3), de préférence sous forme de particules solides.

9. Procédé selon la revendication 7 ou 8, comprenant en alternance des phases de production et des phases de régénération du catalyseur, l'injection de substance étant effectuée lors des phases de réaction et / ou lors des phases de régénération.

10. Procédé selon l'une des revendications 7 à 9, dans lequel la substance est un réactif ou un composé catalytique ou un composé apte à régénérer un catalyseur.

11. Procédé selon l'une des revendications 7 à 10, dans lequel la substance est un gaz comprenant de l'oxygène.

12. Procédé selon l'une des revendications 7 à 11, dans lequel la substance est injectée en plusieurs points du parcours des réactifs dans le compartiment réactionnel (3).

13. Procédé selon l'une des revendications 7 à 12, mis en oeuvre dans un réacteur chimique selon l'une des revendications 1 à 6.

14. Procédé selon l'une des revendications 7 à 13, qui est :
- un procédé de déshydratation du glycérol en acroléine ; ou
- un procédé de déshydratation de l'acide lactique en acide acrylique ; ou
- un procédé de déshydratation de l'acide 3-hydroxypropionique en acide acrylique ; ou
- un procédé de déshydratation de l'acide 3-hydroxyisobutyrique en acide méthacrylique; ou
- un procédé de déshydratation de l'acide 2-hydroxyisobutyrique, en acide methacrylique ; ou
- un procédé de conversion d'un composé chloré en un composé fluoré, de préférence un procédé de préparation d'une hydrofluorooléfine ou d'un hydrofluorocarbure, notamment un procédé de préparation d'un fluoropropène, et de manière tout particulièrement préférée un procédé de préparation du 2,3,3,3-tétrafluoropropène ; ou
- un procédé d'oxydation sélective telle que l'oxydation du méthanol en formaldéhyde ou en diméthoxyméthane ; l'oxydation de l'éthanol en acétaldéhyde ou en diethoxyéthane ; l'oxydation de l'orthoxylène ou du naphtalène en anhydride phtalique ; l'oxydation du benzène, du butène, du butanol ou du butane en anhydride maléique ; l'oxydation du propylène en acroléine ; l'oxydation de l'isobutène ou du tertiobutanol en méthacroléine.

## Patentansprüche

1. Chemischer Reaktor, umfassend mehrere Wärmeaustauschplatten (1), die zwischen sich Reaktionskompartimente (3) definieren, wobei jede Wärmeaustauschplatte (1) zwei Wände (2a, 2b) umfasst, die zwischen sich mindestens einen Wärmeaustauschraum (4) definieren, wobei die jeweiligen Wände (2a, 2b) durch Verbindungszonen (5) aneinander fixiert sind, und der Reaktor außerdem mindestens eine Vorrichtung zum Einleiten von Substanz (6) in die Reaktionskompartimente (3) umfasst, wobei die Substanzeinleitungsvorrichtung (6) die Wärmeaustauschplatten (1) in den jeweiligen Verbindungszonen (5) davon durchquert.

2. Chemischer Reaktor nach Anspruch 1, wobei es sich bei der Substanzeinleitungsvorrichtung (6) um eine Vorrichtung zum Einleiten von Gas und vorzugsweise eine Vorrichtung zum Einleiten von sauerstoffhaltigem Gas handelt.

3. Chemischer Reaktor nach Anspruch 1 oder 2, wobei es sich bei der Substanzeinleitungsvorrichtung (6) um ein Rohr mit mehreren Einleitungsöffnungen handelt.

4. Chemischer Reaktor nach einem der Ansprüche 1 bis 3, wobei die Verbindungszonen (5) in Form von Streifen, vorzugsweise parallelen Streifen, oder in Form von Punkten auf den Wärmeaustauschplatten (1) angeordnet sind.

5. Chemischer Reaktor nach einem der Ansprüche 1 bis 4, umfassend mehrere Substanzeinleitungsvorrichtungen (6), die vorzugsweise parallel zueinander sind und vorzugsweise mit einem Substanzverteilungssystem (8) verbunden sind.

6. Chemischer Reaktor nach einem der Ansprüche 1 bis 5, wobei die Wärmeaustauschplatten (1) in einer Kammer radial oder parallel zueinander angeordnet sind und vorzugsweise in Modulen gruppiert sind.

7. Chemisches Reaktionsverfahren, bei dem man am Eingang eines zwischen zwei Wärmeaustauschplatten (1) definierten Reaktionskompartiments (3) Reaktasten zuführt, am Ausgang des Reaktionskompartiments (3) Reaktionsprodukte abzieht und eine Substanz in das Reaktionskompartiment (3) einleitet, wobei jede Wärmeaustauschplatte (1) zwei Wände (2a, 2b) umfasst, die zwischen sich mindestens einen Wärmeaustauschraum (4) definieren, wobei die jeweiligen Wände (2a, 2b) durch Verbindungszonen (5) aneinander fixiert sind, und das Einleiten von Substanz mit Hilfe mindestens einer Substanzeinleitungsvorrichtung (6), die die Wärmeaustauschplatten (1) in den jeweiligen Verbindungszonen (5) davon durchquert, durchgeführt wird.

8. Verfahren nach Anspruch 7, wobei es sich bei der Reaktion um eine heterogene katalytische Reaktion handelt und ein Katalysator in den Reaktionskompartimenten (3) angeordnet ist, vorzugsweise in Form von festen Teilchen.

9. Verfahren nach Anspruch 7 oder 8 mit alternierenden Produktionsphasen und Katalysatorregenerationsphasen, wobei das Einleiten von Substanz während der Reaktionsphasen und/oder während der Regenerationsphasen durchgeführt wird.

10. Verfahren nach einem der Ansprüche 7 bis 9, wobei es sich bei der Substanz um einen Reaktanten oder eine katalytisch wirksame Verbindung oder eine zur Regenerierung eines Katalysators befähigte Verbindung handelt.

11. Verfahren nach einem der Ansprüche 7 bis 10, wobei es sich bei der Substanz um ein sauerstoffhaltiges Gas handelt.

12. Verfahren nach einem der Ansprüche 7 bis 11, wobei die Substanz an mehreren Punkten des Wegs der Reaktanten in das Reaktionskompartiment (3) eingeleitet wird.

13. Verfahren nach einem der Ansprüche 7 bis 12, das in einem chemischen Reaktor gemäß einem der Ansprüche 1 bis 6 durchgeführt wird.

14. Verfahren nach einem der Ansprüche 7 bis 13, bei dem es sich um:
- ein Verfahren zur Dehydratisierung von Glycerin zu Acrolein; oder
- ein Verfahren zur Dehydratisierung von Milchsäure zu Acrylsäure; oder
- ein Verfahren zur Dehydratisierung von 3-Hydroxypropionsäure zu Acrylsäure; oder
- ein Verfahren zur Dehydratisierung von 3-Hydroxyisobuttersäure zu Methacrylsäure; oder
- ein Verfahren zur Dehydratisierung von 2-Hydroxyisobuttersäure zu Methacrylsäure; oder
- ein Verfahren zur Umwandlung einer Chlorverbindung in eine Fluorverbindung, vorzugsweise ein Verfahren zur Herstellung eines teilfluorierten Fluorolefins oder eines teilfluorierten Fluorkohlenwasserstoffs, insbesondere ein Verfahren zur Herstellung eines Fluorpropens und ganz besonders bevorzugt ein Verfahren zur Herstellung von 2,3,3,3-Tetrafluorpropen; oder
- ein Verfahren zur selektiven Oxidation wie die Oxidation von Methanol zu Formaldehyd oder zu Dimethoxymethan; die Oxidation von Ethanol zu Acetaldehyd oder zu Dimethoxyethan; die Oxidation von ortho-Xylol oder Naphthalin zu Phthalsäureanhydrid; die Oxidation von Benzol, Buten, Butanol oder Butan zu Maleinsäureanhydrid; die Oxidation von Propylen zu Acrolein; die Oxidation von Isobutylen oder tert.-Butanol zu Methacrolein
handelt.

## Claims

1. A chemical reactor comprising a plurality of heat-exchange plates (1) defining, between them, reaction compartments (3), in which reactor each heat-exchange plate (1) comprises two walls (2a, 2b) defining, between them, at least one heat-exchange space (4), the respective walls (2a, 2b) being fixed to one another by joining regions (5), and the reactor also comprises at least one device for injection of substance (6) into the reaction compartments (3), said substance-injecting device (6) passing through the heat-exchange plates (1) in respective joining regions (5) of the plates.

2. The chemical reactor as claimed in claim 1, in which the substance-injecting device (6) is a device for the injection of gas and preferably a device for the injection of oxygen-comprising gas.

3. The chemical reactor as claimed in claim 1 or 2, in which the substance-injecting device (6) is a pipe exhibiting a plurality of injection orifices.

4. The chemical reactor as claimed in one of claims 1 to 3, in which the joining regions (5) are positioned on the heat-exchange plates (1) in the form of strips, preferably parallel strips, or in the form of points.

5. The chemical reactor as claimed in one of claims 1 to 4, comprising a plurality of substance-injecting devices (6), preferably parallel to one another and preferably connected to a substance-distributing system (8).

6. The chemical reactor as claimed in one of claims 1 to 5, in which the heat-exchange plates (1) are positioned in a chamber in a radial manner or in a manner parallel to one another and are preferably grouped into modules.

7. A chemical reaction process comprising the admission of reactants at the inlet of a reaction compartment (3) defined between two heat-exchange plates (1), the withdrawal of reaction products at the outlet of the reaction compartment (3), and the injection of a substance into the reaction compartment (3), in which process each heat-exchange plate (1) comprises two walls (2a, 2b) defining, between them, at least one heat-exchange space (4), the respective walls (2a, 2b) being fixed to one another by joining regions (5), and the injection of substance being carried out by means of at least one substance-injecting device (6) which passes through the heat-exchange plates (1) in respective joining regions (5) of the plates.

8. The process as claimed in claim 7, in which the reaction is of the heterogeneous catalytic type and a catalyst is positioned in the reaction compartments (3), preferably in the form of solid particles.

9. The process as claimed in claim 7 or 8, alternately comprising phases of production and phases of regeneration of the catalyst, the injection of substance being carried out during the reaction phases and/or during the regeneration phases.

10. The process as claimed in one of claims 7 to 9, in which the substance is a reactant or a catalytic compound or a compound capable of regenerating a catalyst.

11. The process as claimed in one of claims 7 to 10, in which the substance is an oxygen-comprising gas.

12. The process as claimed in one of claims 7 to 11, in which the substance is injected at several points of the route of the reactants in the reaction compartment (3).

13. The process as claimed in one of claims 7 to 12, carried out in a chemical reactor as claimed in one of claims 1 to 6.

14. The process as claimed in one of claims 7 to 13, which is:
- a process for the dehydration of glycerol to give acrolein; or
- a process for the dehydration of lactic acid to give acrylic acid; or
- a process for the dehydration of 3-hydroxypropionic acid to give acrylic acid; or
- a process for the dehydration of 3-hydroxyisobutyric acid to give methacrylic acid; or
- a process for the dehydration of 2-hydroxyisobutyric acid to give methacrylic acid; or
- a process for the conversion of a chlorinated compound into a fluorinated compound, preferably a process for the preparation of a hydrofluoroolefin or of a hydrofluorocarbon, in particular a process for the preparation of a fluoropropene and very particularly preferably a process for the preparation of 2,3,3,3-tetrafluoropropene; or
- a selective oxidation process, such as the oxidation of methanol to give formaldehyde or dimethoxymethane; the oxidation of ethanol to give acetaldehyde or diethoxyethane; the oxidation of ortho-xylene or naphthalene to give phthalic anhydride; the oxidation of benzene, butene, butanol or butane to give maleic anhydride; the oxidation of propylene to give acrolein; or the oxidation of isobutene or tert-butanol to give methacrolein.
